# EUROPÄISCHE PATENTSCHRIFT

(11) **EP 1 282 608 B1**
(45) Veröffentlichungstag und Bekanntmachung des Hinweises auf die Patenterteilung: **20.08.2003**
(21) Anmeldenummer: 01940365.8
(22) Anmeldetag: 26.04.2001
(51) Int. Cl.: C07D 241/12

(54) **HERSTELLUNG VON 2-ETHYL-3-METHYL-1,4-DIAZIN**
PRODUCTION OF 2-ETHYL-3-METHYL-1,4-DIAZINE
PREPARATION DE 2-ETHYL-3-METHYL-1,4-DIAZINE

(30) Priorität: 08.05.2000 DE 10022361
(43) Veröffentlichungstag der Anmeldung: 12.02.2003
(73) Patentinhaber: Haarmann & Reimer GmbH, 37603 Holzminden (DE)
(72) Erfinder: LAMBRECHT, Stefan, 37603 Holzminden (DE)
(74) Vertreter: Eisenführ, Speiser & Partner
(86) Internationale Anmeldenummer: EP0104697
(87) Internationale Veröffentlichungsnummer: WO01085703

(56) Entgegenhaltungen:
- US-A- 3 702 253
- CHEMICAL ABSTRACTS, vol. 104, no. 19, 12. Mai 1986 (1986-05-12) Columbus, Ohio, US; abstract no. 168437, TSAI, SONGCHUAN ET AL: "Syntheses of pyrazines. I" XP002176855 in der Anmeldung erwähnt & NANJING DAXUE XUEBAO, ZIRAN KEXUE (1984), (2), 245-9, 2 PLATES,
- CHEMICAL ABSTRACTS, vol. 52, no. 14, 25. Juli 1958 (1958-07-25) Columbus, Ohio, US; abstract no. 11862a, XP002176856 & TAKEO ISHIGURO: "Syntheses of piperazines. VIII" YAKUGAKU ZASSHI, Bd. 78, 1958, Seiten 229-231,
- RIZZI G. P.: "Some reactions of methyl- pyrazines with organolithium reagents" JOURNAL OF ORGANIC CHEMISTRY., Bd. 33, Nr. 4, April 1968 (1968-04), Seiten 1333-1337, XP002176853 AMERICAN CHEMICAL SOCIETY. EASTON., US ISSN: 0022-3263
- CHEMICAL ABSTRACTS, vol. 88, no. 15, 10. April 1978 (1978-04-10) Columbus, Ohio, US; abstract no. 105412, ENOMOTO, YOSHIYUKI ET AL: "Pyrazine derivatives" XP002176857 in der Anmeldung erwähnt & JP 52 136182 A (OGAWA AND CO., LTD., JAPAN) 14. November 1977 (1977-11-14)
- NAKATANI Y. ET AL.: "Synthèse facile de pyrazines disubstituées en 2 et 3" AGRICULTURAL AND BIOLOGICAL CHEMISTRY., Bd. 37, Nr. 6, 1973, Seiten 1509-1510, XP001024034 JAPAN SOC. FOR BIOSCIENCE, BIOTECHNOLOGY AND AGROCHEM. TOKYO., JP ISSN: 0002-1369 in der Anmeldung erwähnt

## Beschreibung

Die Erfindung betrifft ein neues, industrielles Herstellungsverfahren für 2-Ethyl-3-methyl-1,4-diazin bei dem auf umwelt- und sicherheitsproblematische Lösungsmittel verzichtet werden kann, nur wenige Verfahrensschritte benötigt werden und der Einsatz an Hilfsstoffen wie z.B. Lösungsmitteln in diesem Prozess minimal ist.

2-Ethyl-3-methyl-1,4-diazin ist ein wichtiger Aromastoff (Allured's Flavor and Fragrance Materials - 1995, Allured Publishing Corporation, 1995, S. 127). Ein technisches Verfahren, dass auf einfache und kostengünstige Weise 2-Ethyl-3-methyl-1,4-diazin liefert, ist deshalb von großem wirtschaftlichem Interesse.

Die Herstellung von 2-Ethyl-3-methyl-1,4-diazin ist an sich bekannt. Die Herstellung erfolgt ausgehend von 2,3-Pentandion und 1,2-Ethandiamin in einem zweistufigen Prozess und wird durch das folgende Schema verdeutlicht:

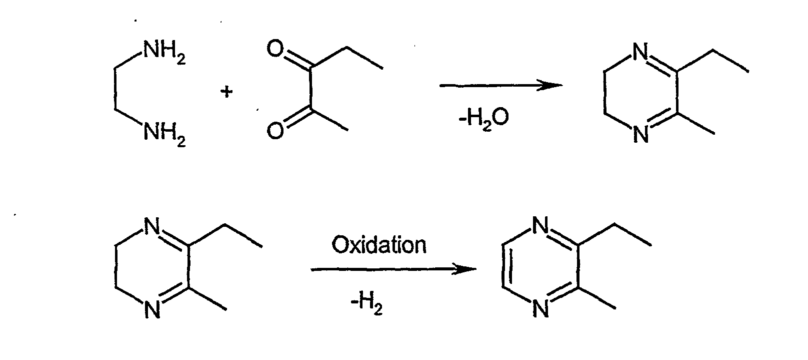

Die bekannten Syntheseverfahren sind aber für eine industrielle Umsetzung ungeeignet. Insbesondere müssen folgende Kriterien für ein technisches Herstellverfahren erfüllt sein:
- Verwendung von unter Sicherheits- und Umweltaspekten unproblematischer Lösungsmittel
- wenige Verfahrensschritte
- eine hohe Raum-Zeit-Ausbeute
- gefahrlose Durchführung der Reaktion

Die bekannten Herstellverfahren erfüllen diese Bedingungen nicht. Es werden beispielweise umweltkritische Oxidationsmittel wie Kupferchromit (*Helv. Chim. Acta* 1967, *50*, 1754) verwendet. Der Umgang mit umweltkritischen Stoffen erfordert eine aufwendige Reaktionsführung und sichere Deponie der Reststoffe. Als Lösungsmittel werden Gefahrstoffe wie Ethylenglycol (*Agr. Biol. Chem*. 1973, *37*, 1509) verwendet. So liegt bei Ethylenglycol die maximale Arbeitsplatzkonzentration bei 10 ppm (MAK- und BAT-Werte-Liste, 1999, Wiley-VCH, Weinheim). Die Anzahl der einzelnen Verfahrensschritte ist hoch (*Nanjing Daxue Xuebo, Ziran Kexue* 1984, 245 und obige Autoren). Nach dem Verfahren der JP 52 136 182 wird 2-Ethyl-3-methyl-1,4-diazin ebenfalls in Gegenwart ökologisch bedenklicher Oxidationsmittel wie Manganoxid, Kupferoxid oder Bleioxid hergestellt. Zudem wird eine große Menge dieser Oxidationsmittel verwendet, was die Herstellung schwierig und in der Durchführung teuer macht.

Ein technisches Verfahren, dass auf einfache und kostengünstige Weise 2-Ethyl-3-methyl-1,4-diazin liefert, ist deshalb von großem wirtschaftlichen Interesse.

Es wurde ein Verfahren zur Herstellung von 2-Ethyl-3-methyl-1,4-diazin gefunden, dadurch gekennzeichnet, dass 2,3-Pentandion und 1,2-Ethandiamin in einem aliphatischen Monoalkohol, Diol oder Triol mit drei bis acht Kohlenstoffatomen als Lösungsmittel unter Zugabe katalytischer Mengen einer alkalisch reagierenden Substanz umgesetzt werden und dann in eine Lösung einer alkalisch reagierenden Verbindung in einem aliphatischen Monoalkohol, Diol oder Triol mit drei bis acht Kohlenstoffatomen und einem dehydrierend wirkenden Katalysator, der auf einem Träger aufgetragen sein kann, eingetropft wird und die Abtrennung des Produktes durch Wasserdampfdestillation erfolgt.

Mit der vorliegenden Erfindung gelingt es, die genannten Nachteile zu überwinden und ein technisch günstiges Verfahren bereit zu stellen.

Aliphatische Monoalkohole, Diole oder Triole sind beispielweise Butanol, Pentanol, Hexanol, Heptanol, Octanol, 1,2-Butandiol, 1,4-Butandiol, 1,3-Propandiol, 1,2-Propandiol und Glycerin.

Bevorzugte aliphatische Monoalkohole, Diole oder Triole sind 1,3-Propandiol, 1,2-Propandiol und Glycerin.

Besonders bevorzugt ist 1,2-Propandiol.

Die alkalisch reagierende Substanz kann beispielsweise ein Alkalicarbonat, ein Alkalihydrogencarbonat oder Alkaliacetat sein.

Bevorzugte alkalisch reagierende Substanzen sind Alkalicarbonate.

Eine besonders bevorzugte alkalisch reagierende Substanz ist Kaliumcarbonat.

Dehydrierend wirkende Katalysatoren im erfindungsgemäßen Sinne enthalten Elemente der 1., 2., 6., 7. oder 8. Nebengruppe, deren Verbindungen, Oxide, Legierungen oder Mischungen. Diese Katalysatoren können beispielsweise in fein verteilter Form, aufgebracht auf Träger oder zusammen mit anderen Metallen oder anderen Metallverbindungen eingesetzt werden. Die dehydrierend wirkenden Katalysatoren können Dotierungen mit einem oder mehreren beliebigen Metallen oder deren Verbindungen enthalten.

Geeignete dehydrierend wirkende Katalysatoren können beispielsweise Kupfer, Silber, Zink, Chrom, Molybdän, Wolfram, Mangan, Rhenium, Eisen, Ruthenium, Osmium, Kobalt, Rhodium, Iridium, Nickel, Palladium oder Platin enthalten.

Besonders vorteilhaft sind dehydrierend wirkende Katalysatoren enthaltend die Elemente Palladium, Platin, Ruthenium oder Rhodium.

Ganz besonders bevorzugter dehydrierend wirkender Katalysator ist Palladium, das auf einem Trägermaterial aufgetragen sein kann.

Die erfindungsgemäßen deyhdrierend wirkenden Katalysatoren können auf organische oder anorganische Trägermaterialien aufgebracht sein. Als vorteilhafte Trägermaterialen seien genannt Aktivkohle, Kohle, Aluminiumoxide, Metalloxide, Kieselgele, Zeolithe, Tone, Tongranulate, amorphe Aluminiumsilicate, oder sonstige anorganische oder polymere Träger. Bevorzugtes Trägermaterial ist Aktivkohle.

Für das erfindungsgemäße Verfahren werden im allgemeinen dehydrierend wirkende Katalysatoren auf einem Trägermaterial eingesetzt, wobei der Gewichtsanteil des Elements der 1., 2., 6., 7. oder 8. Nebengruppe 1 bis 15 Gew.-%, bevorzugt 5 bis 10 Gew.-%, des Katalysatorgewichtes beträgt.

Alkalisch reagierende Verbindungen können beispielsweise Alkalialkoholate, Alkalihydroxide oder Erdalkalihydroxide sein. Bevorzugt sind Kaliumhydroxid, Natriumhydroxid, Kaliummethylat, Kaliumethylat, Natriummethylat und Natriumethylat.

Das erfindungsgemäße Verfahren ist im Vergleich zu den bekannten vorteilhaft. Es kann mit problemlosen Lösungsmitteln wie zum Beispiel 1,2-Propandiol, das für die Verwendung in Lebensmitteln zugelassen ist, gearbeitet werden. Es ist dabei überraschend, dass die Menge an Lösungsmittel in dem ersten Verfahrensschritt ohne Ausbeuteminderung sehr gering sein kann. Hierdurch wird die Raum-Zeit-Ausbeute erhöht und die Wirtschaftlichkeit des Verfahrens damit verbessert. Es ist zudem überraschend, dass das Lösungsmittel nach der Reaktion zu der Zwischenstufe nicht entfernt werden muss. Das Reaktionsgemisch kann direkt und ohne weitere Reinigungs- oder Isolierungsoperationen in der zweiten Reaktion eingesetzt werden. Die Verwendung eines erfindungsgemäßen Lösungsmittels wie z.B. 1,2-Propandiol ermöglicht zudem die einfache und wirtschaftliche Isolierung des Produktes, da 2-Ethyl-3-methyl-1,4-diazin in einer Wasserdampfdestillation vom Reaktionsgemisch abgetrennt werden kann. Andere Alkohole wie Ethanol (*Agr. Biol. Chem.* 1973, *37*, 1509) haben zu geringe Siedepunkte, so dass eine Wasserdampfdestillation unmöglich ist. Weitere höhere Alkohole haben zu geringe Lösungseigenschaften, so dass deren Verwendung ebenfalls unvorteilhaft ist.

Es ist zudem überraschend, dass bereits der Zusatz von katalytischen Mengen der erfindungsgemäßen alkalisch reagierenden Substanzen ausreicht, um das entstehende Zwischenprodukt zu stabilisieren. Dies ist besonders vorteilhaft, da geringe Mengen der erfindungsgemäßen alkalisch reagierenden Substanzen in Lösung bleiben und somit die Handhabung stark vereinfacht ist.

Es ist weiterhin überraschend, dass das Reaktionsgemisch aus dem ersten Verfahrensschritt vorteilhaft in eine Lösung der erfindungsgemäßen alkalisch reagierenden Verbindungen in einem erfindungsgemäßen aliphatischen Diol oder Triol mit drei bis acht Kohlenstoffatomen und einem erfindungsgemäßen deyhdrierend wirkenden Katalysator auf einem Träger eingetropft werden kann. Da bei der Reaktion Gas entsteht, wird auf diese Weise die spontane Freisetzung einer großen Gasmenge vermieden. Dies erhöht die Sicherheit des Verfahrens stark.

Der erste Schritt des erfindungsgemäßen Verfahrens wird im allgemeinen im Temperaturbereich von -10 bis 100°C, bevorzugt bei 10 bis 60°C, durchgeführt. Die Einsatzmaterialien 2,3-Pentandion und 1,2-Ethandiamin können zu gleichen molaren Mengen oder im Unter- bzw. Überschuß zugegeben werden. Bevorzugt ist ein molares Verhältnis von 2,3-Pentandion zu 1,2-Ethandiamin von 0,1 zu 1 bis 2 zu 1. Die Menge der erfindungsgemäßen alkalisch reagierenden Substanz kann 0,0001 bis 40 Gew.%, vorzugsweise 0,001 bis 5 Gew.%, bezogen auf 2,3-Pentandion betragen.

Als Lösungsmittel wird ein aliphatisches Diol oder Triol mit drei bis acht Kohlenstoffatomen, bevorzugt 1,2-Propandiol, verwendet. Die Menge an Lösungsmittel kann zwischen 10 und 300 Gew.-%, bezogen auf 2,3-Pentandion betragen.

Der zweite Schritt des erfindungsgemäßen Verfahrens wird im allgemeinen im Temperaturbereich von 70 bis 180°C, bevorzugt bei 80 bis 130°C, durchgeführt. Das Reaktionsgemisch der ersten Stufe wird bei dieser Temperatur in eine Lösung einer erfindungsgemäßen alkalisch reagierenden Verbindung in einem erfindungsgemäßen Diol oder Triol mit drei bis acht Kohlenstoffatomen und einem dehydrierend wirkenden Katalysator eingetropft. Die Katalysatormenge kann 0,0001 bis 1 Gew.-%, vorzugsweise 0,001 bis 0,1 Gew.%, bezogen auf eingesetztes 2,3-Pentandion, betragen. Die Menge der alkalisch reagierenden Verbindung kann zwischen 0,2 und 3 Moläquivalenten, bevorzugt zwischen 0,4 und 1,5 Moläquivalenten bezogen auf 2,3-Pentandion, liegen.

Das Verfahren kann unter Normaldruck und unter Über- oder Unterdruck durchgeführt werden. Beispielsweise sei der folgende Druckbereich genannt: 0,1 bis 10 bar, bevorzugt 0,2 bis 5 bar.

Zur Abtrennung des 2-Ethyl-3-methyl-1,4-diazin vom Reaktionsgemisch wird nach dem erfindungsgemäßen Verfahren eine Wasserdampfdestillation unter Normaldruck oder im Vakuum, bevorzugt im Vakuum, besonders bevorzugt bei einem Vakuum von 10 bis 500 mbar, durchgeführt.

### Beispiel 1

### Herstellung von 2-Ethyl-3-methyl-1,4-diazin mit Kaliumhydroxid als alkalisch reagierender Verbindung

### Erster Verfahrensschritt:

100 ml 1,2-Propandiol und 0,5 g Kaliumcarbonat wurden vorgelegt und auf 0°C gekühlt. Bei dieser Temperatur wurden 247 g 2,3-Pentandion und 158 g 1,2-Ethandiamin zugetropft (Reaktionsgemisch erste Stufe).

### Zweiter Verfahrensschritt:

200 ml 1,2-Propandiol, 90 g Kaliumhydroxid und 0,8 g Palladium auf Aktivkohle, 5 Gew.-% vom Katalysatorgewicht, wurden gemischt und auf 100°C erhitzt. Bei dieser Temperatur wurde das Reaktionsgemisch erste Stufe zugetropft. Die Nachreaktionszeit betrug 3 Stunden.

Das Produkt wurde durch Wasserdampfdestillation bei 125 mbar vom Reaktionsgemisch abgetrennt. Das Destillat wurde mit Natiumchlorid gesättigt und mit Methyltert.-butylether extrahiert.

Es wurde 2-Ethyl-3-methyl-1,4-diazin in einer Ausbeute von 56 % erhalten.
Siedepunkt: 61°C (15 mbar).

### Beispiel 2

### Herstellung von 2-Ethyl-3-methyl-1,4-diazin mit Natriummethylat als alkalisch reagierender Verbindung.

### Erster Verfahrensschritt:

42 g 1,2-Propandiol und 0,21 g Kaliumcarbonat wurden vorgelegt und bei 20°C wurden 113 g 2,3-Pentandion und 65,5 g 1,2-Ethandiamin zugetropft (Reaktionsgemisch erste Stufe).

### Zweiter Verfahrensschritt:

184 g 1,2-Propandiol, 100 g Natriummethylat und 0,32 g Palladium auf Aktivkohle, 5 Gew.-% vom Katalysatorgewicht, wurden gemischt und auf 100°C erhitzt. Bei dieser Temperatur wurde das Reaktionsgemisch erste Stufe zugetropft. Die Nachreaktionszeit betrug 3 Stunden.

Das Produkt wurde durch Wasserdampfdestillation bei 130 mbar vom Reaktionsgemisch abgetrennt. Das Destillat wurde mit Natiumchlorid gesättigt und mit Methyltert.-butylether extrahiert.

Es wurde 2-Ethyl-3-methyl-1,4-diazin in einer Ausbeute von 60 % erhalten.
Siedepunkt: 61°C (15 mbar).

### Beispiel 3

### Herstellung von 2-Ethyl-3-methyl-1,4-diazin in Gegenwart verschiedener dehydrierend wirkender Katalysatoren (siehe Tabelle 1).

### Erster Verfahrensschritt:

41 g 1,2-Propandiol und 0,21g Kaliumcarbonat wurden bei 20°C vorgelegt. Bei dieser Temperatur wurden 100 g 2,3-Pentandion und 65,2 g 1,2-Ethandiamin zugetropft. (Reaktionsgemisch erste Stufe).

### Zweiter Verfahrensschritt:

82 g 1,2-Propandiol, 36,7 g Kaliumhydroxid und die entsprechende Menge des deyhdrierend wirkenden Katalysators (siehe Tabelle 1) wurden gemischt und auf die angegebenen Temperatur erhitzt. Bei dieser Temperatur wurde das Reaktionsgemisch erste Stufe zugetropft. Die Nachreaktionszeit betrug 2 Stunden.

Das Produkt wurde durch Wasserdampfdestillation bei 130 mbar vom Reaktionsgemisch abgetrennt. Das Destillat wurde mit Natriumchlorid gesättigt und mit Methyltert.-butylether extrahiert.

Siedepunkt: 61°C (15mbar).

Die in Tabelle 1 angegebenen prozentualen Katalysatorangaben entsprechen den Gewichstangaben der Elemente bezogen auf das Gesamtkatalysatorgewicht.

**Tabelle 1:**

| Katalysator | Reaktions-temperatur | Ausbeute |
|---|---|---|
| 1,55 g Pt auf Aktivkohle (5 Gew.-%) | 130°C | 21 % |
| 0,93 g Pd auf Aluminiumoxid (5 Gew.-%) | 120°C | 54 % |
| 1,24 g Pd auf Aluminiumoxid (5 Gew.-%) | 120°C | 53 % |
| 2 g aktivierter Nickelkatalysator, Molybdän dotiert | 120°C | 30 % |
| 2 g aktivierter Nickelkatalysator | 120°C | 29 % |
| 0,62 g Raney-Nickel | 120°C | 32 % |
| 1,0 g Pt/Rh auf Aktivkohle (5 Gew.-%) | 120°C | 31 % |

## Patentansprüche

1. Verfahren zur Herstellung von 2-Ethyl-3-methyl-1,4-diazin, **dadurch gekennzeichnet dass** 2,3-Pentandion und 1,2-Ethandiamin in einem aliphatischen Monoalkohol, Diol oder Triol mit drei bis acht Kohlenstoffatomen als Lösungsmittel unter Zugabe katalytischer Mengen einer alkalisch reagierenden Substanz umgesetzt werden und die Reaktionsmischung dann in eine Lösung einer alkalisch reagierenden Verbindung in einem aliphatischen Monoalkohol, Diol oder Triol mit drei bis acht Kohlenstoffatomen und einem dehydrierend wirkenden Katalysator, der auf einem Träger aufgetragen sein kann, eingetropft wird und die Abtrennung des Produktes durch Wasserdampfdestillation erfolgt.

2. Verfahren nach Anspruch 1, **dadurch gekennzeichnet, dass** das Lösungsmittel Butanol, Pentanol, Hexanol, Heptanol, Octanol, 1,2-Butandiol, 1,4-Butandiol, 1,3-Propandiol, 1,2-Propandiol oder Glycerin ist.

3. Verfahren nach Anspruch 2, **dadurch gekennzeichnet, dass** das Lösungsmittel 1,3-Propandiol, 1,2-Propandiol oder Glycerin ist.

4. Verfahren nach Anspruch 3, **dadurch gekennzeichnet, dass** das Lösungsmittel 1,2-Propandiol ist.

5. Verfahren nach Anspruch 1, **dadurch gekennzeichnet, dass** die Menge an Lösungsmittel im ersten Verfahrensschritt zwischen 10 und 300 Gew.-%, bezogen auf 2,3-Pentandion, beträgt.

6. Verfahren nach Anspruch 1, **dadurch gekennzeichnet, dass** die alkalisch reagierende Substanz ein Alkalicarbonat, ein Alkalihydrogencarbonat oder Alkaliacetat ist.

7. Verfahren nach Anspruch 6, **dadurch gekennzeichnet, dass** die alkalisch reagierende Substanz ein Alkalicarbonat ist.

8. Verfahren nach Anspruch 7, **dadurch gekennzeichnet, dass** die alkalisch reagierende Substanz Kaliumcarbonat ist.

9. Verfahren nach Anspruch 1, **dadurch gekennzeichnet, dass** die Menge der alkalisch reagierenden Substanz zwischen 0,0001 bis 40 Gew.-% bezogen auf 2,3-Pentandion beträgt.

10. Verfahren nach Anspruch 1, **dadurch gekennzeichnet, dass** der erste Verfahrensschritt bei -10 bis 100°C durchgeführt wird.

11. Verfahren nach Anspruch 1, **dadurch gekennzeichnet, dass** der zweite Verfahrensschritt bei 70 bis 180°C durchgeführt wird.

12. Verfahren nach Anspruch 1, **dadurch gekennzeichnet, dass** der dehydrierend wirkende Katalysator die Elemente Kupfer, Silber, Zink, Chrom, Molybdän, Wolfram, Mangan, Rhenium, Eisen, Ruthenium, Osmium, Kobalt, Rhodium, Iridium, Nickel, Palladium oder Platin enthält.

13. Verfahren nach Anspruch 1, **dadurch gekennzeichnet, dass** die alkalisch reagierende Verbindung ein Alkalialkoholat, Alkalihydroxid oder Erdalkalihydroxid ist.

## Claims

1. Process for the production of 2-ethyl-3-methyl-1,4-diazine, **characterised in that** 2,3-pentanedione and 1,2-ethanediamine are reacted in an aliphatic monoalcohol, diol or triol having three to eight carbon atoms, as solvent, with the addition of catalytic amounts of a substance having an alkaline reaction and the reaction mixture is then added dropwise to a solution of a compound having an alkaline reaction in an aliphatic monoalcohol, diol or triol having three to eight carbon atoms and a catalyst having a dehydrogenating action, which can be applied to a support, and the product is separated off by steam distillation.

2. Process according to Claim 1, **characterised in that** the solvent is butanol, pentanol, hexanol, heptanol, octanol, 1,2-butanediol, 1,4-butanediol, 1,3-propanediol, 1,2-propanediol or glycerol.

3. Process according to Claim 2, **characterised in that** the solvent is 1,3-propanediol, 1,2-propanediol or glycerol.

4. Process according to Claim 3, **characterised in that** the solvent is 1,2-propanediol.

5. Process according to Claim 1, **characterised in that** the amount of solvent in the first process step is between 10 and 300% (m/m), based on 2,3-pentanedione.

6. Process according to Claim 1, **characterised in that** the substance having an alkaline reaction is an alkali metal carbonate, an alkali metal bicarbonate or alkali metal acetate.

7. Process according to Claim 6, **characterised in that** the substance having an alkaline reaction is an alkali metal carbonate.

8. Process according to Claim 7, **characterised in that** the substance having an alkaline reaction is potassium carbonate.

9. Process according to Claim 1, **characterised in that** the amount of the substance having an alkaline reaction is between 0.0001 and 40% (m/m), based on 2,3-pentanedione.

10. Process according to Claim 1, **characterised in that** the first process step is carried out at -10 to 100°C.

11. Process according to Claim 1, **characterised in that** the second process step is carried out at 70 to 180°C.

12. Process according to Claim 1, **characterised in that** the catalyst having a dehydrogenating action contains the elements, copper, silver, zinc, chromium, molybdenum, tungsten, manganese, rhenium, iron, ruthenium, osmium, cobalt, rhodium, iridium, nickel, palladium or platinum.

13. Process according to Claim 1, **characterised in that** the compound having an alkaline reaction is an alkali metal alcoholate, alkali metal hydroxide or alkaline earth metal hydroxide.

## Revendications

1. Procédé de préparation de la 2-éthyl-3-méthyl-1,4-diazine **caractérisé en ce que** la 2,3-pentanedione et la 1,2-éthanediamine sont mises à réagir dans un monoalcool, diol ou triol aliphatique ayant 3 à 8 atomes de carbone comme solvant avec addition de quantités catalytiques d'une substance à réaction alcaline, après quoi le mélange réactionnel est introduit goutte à goutte dans une solution d'un composé à réaction alcaline dans un monoalcool, diol ou triol aliphatique ayant 3 à 8 atomes de carbone et un catalyseur à action déshydrogénante, qui peut être appliqué sur un support, et la séparation du produit a lieu par entraînement à la vapeur d'eau.

2. Procédé selon la revendication 1 **caractérisé en ce que** le solvant est le butanol, le pentanol, l'hexanol, l'heptanol, l'octanol, le 1,2-butanediol, le 1,4-butanediol, le 1,3-propanediol, le 1,2-propanediol ou la glycérine.

3. Procédé selon la revendication 2 **caractérisé en ce que** le solvant est le 1,3-propanediol, le 1,2-propanediol ou la glycérine.

4. Procédé selon la revendication 3 **caractérisé en ce que** le solvant est le 1,2-propanediol.

5. Procédé selon la revendication 1 **caractérisé en ce que** la quantité de solvant dans la première étape du procédé est de 10 à 300 % en masse par rapport à la 2,3-pentanedione.

6. Procédé selon la revendication 1 **caractérisé en ce que** la substance à réaction alcaline est un carbonate alcalin, un hydrogénocarbonate alcalin ou un acétate alcalin.

7. Procédé selon la revendication 6 **caractérisé en ce que** la substance à réaction alcaline est un carbonate alcalin.

8. Procédé selon la revendication 7 **caractérisé en ce que** la substance à réaction alcaline est le carbonate de potassium.

9. Procédé selon la revendication 1 **caractérisé en ce que** la quantité de substance à réaction alcaline est de 0,0001 à 40 % en masse par rapport à la 2,3-pentanedione.

10. Procédé selon la revendication 1 **caractérisé en ce que** la première étape du procédé est accomplie à -10 à 100°C.

11. Procédé selon la revendication 1 **caractérisé en ce que** la deuxième étape du procédé est accomplie à 70 à 180°C.

12. Procédé selon la revendication 1 **caractérisé en ce que** le catalyseur à action déshydrogénante contient les éléments cuivre, argent, zinc, chrome, molybdène, tungstène, manganèse, rhénium, fer, ruthénium, osmium, cobalt, rhodium, iridium, nickel, palladium ou platine.

13. Procédé selon la revendication 1 **caractérisé en ce que** le composé à réaction alcaline est un alcoolate alcalin, un hydroxyde alcalin ou un hydroxyde alcalino-terreux.
